# FASCICULE DE BREVET EUROPEEN

(11) **EP 1 542 795 B1**
(45) Date de publication et mention de la délivrance du brevet: **14.02.2007**
(21) Numéro de dépôt: 03773807.7
(22) Date de dépôt: 23.09.2003
(51) Int. Cl.: B01J 27/182, B01J 27/186, B01J 23/30, B01J 21/18, C07C 319/04, C07B 45/06, C07C 319/00, C07C 321/04

(54) **PROCEDE CATALYTIQUE DE FABRICATION DE MERCAPTANS A PARTIR DE THIOETHERS**
KATALYTISCHES VERFAHREN ZUR HERSTELLUNG VON MERCAPTANEN AUS THIOETHEREN
CATALYTIC METHOD OF PRODUCING MERCAPTANS FROM THIOETHERS

(30) Priorité: 25.09.2002 FR 0211922
(43) Date de publication de la demande: 22.06.2005
(73) Titulaire: Arkema France, 92800 Puteaux (FR)
(72) Inventeur: FREMY, Georges, F-64390 Sauveterre de Bearn (FR); ESSAYEM, Nadine, F-38540 Saint-Just-Chaleyssin (FR); LACROIX, Michel, F-69009 Lyon (FR); ZAUSA, Elodie, F-33760 Targon (FR)
(74) Mandataire: Treuil, Claude
(86) Numéro de dépôt international: PCT/FR2003/002790
(87) Numéro de publication internationale: WO 2004/029022

(56) Documents cités:
- US-A- 2 402 614
- US-A- 4 005 149
- US-A- 4 059 636
- US-A- 4 927 972

## Description

La présente invention concerne le domaine des mercaptans (encore appelés thiols) et a plus particulièrement pour objet un procédé catalytique pour la fabrication de mercaptans à partir de thioéthers et d'hydrogène sulfuré, en présence d'hydrogène et d'un catalyseur spécifique.

L'intérêt industriel des mercaptans ou thiols fait que de nombreux travaux ont été effectués en vue de la mise au point de la fabrication de ces composés. On connaît notamment un procédé largement employé qui met en oeuvre la réaction de l'hydrogène sulfuré avec un alcool ou une oléfine. Dans une telle réaction, on obtient en particulier comme sous-produit un ou plusieurs thioéthers qui résultent de réactions secondaires, et principalement de la réaction du mercaptan (formé dans la réaction principale) sur le réactif de départ c'est-à-dire soit l'alcool, soit l'oléfine, selon le procédé utilisé.

Les thioéthers obtenus en tant que sous-produits lors de la fabrication de mercaptans n'ont généralement pas d'intérêt commercial.

Des méthodes de conversion de ces thioéthers en vue de leur valorisation ont été proposées, visant à les transformer en mercaptans par réaction avec l'hydrogène sulfuré (H₂S) en présence de différents catalyseurs, réaction dite de sulfhydrolyse.

Les procédés existants de sulfhydrolyse mettent en oeuvre cette réaction sous pression en utilisant un flux réactionnel constitué exclusivement d'H₂S et de thioéther en différentes proportions, en présence de différents systèmes catalytiques.

Le brevet US 4,005,149 décrit ainsi l'obtention de mercaptans (ou thiols) par réaction de l'H₂S sur des sulfures organiques (autre dénomination des thioéthers) en présence, comme catalyseur, d'un sulfure d'un métal du groupe VI et/ou d'un métal du groupe VIII, notamment d'un sulfure de cobalt et de molybdène (Co/Mo) imprégné sur un support d'alumine. Du disulfure de carbone CS₂ est ajouté au mélange réactionnel pour améliorer la conversion du sulfure organique en mercaptan.

Dans le brevet US 4,396,778 est décrit un procédé en phase vapeur de préparation d'alkyl mercaptans à haut poids moléculaire C₁-C₁₈ en utilisant comme catalyseur une zéolithe à larges pores modifiée par du potassium ou du sodium. La réaction est menée à une température élevée, supérieure à 290°C.

Les brevets US 2,829,171 et US 3,081,353 décrivent la synthèse de mercaptans plus légers tel le méthyl mercaptan en présence d'alumine activée comme catalyseur. Les températures de réaction mises en oeuvre dans ces procédés sont élevées.

Des résines échangeuses d'ions fortement acides telles que décrites dans le brevet US 4,927,972 sont des catalyseurs également employés dans les procédés de sulfhydrolyse de thioéthers, mais elles conduisent généralement à un faible rendement.

Le brevet US 4,059,636 décrit l'utilisation d'un catalyseur solide comprenant un acide 12-phosphotungstique supporté sur alumine. Ce catalyseur, comparé à un catalyseur usuel tel qu'un molybdène et cobalt supporté sur alumine (CoMo/Al₂O₃) a pour effet une conversion et une sélectivité plus élevées quand il est mis en oeuvre dans la réaction de sulfhydrolyse, et ce pour une température de réaction plus faible. Il peut toutefois nécessiter la présence de sulfure de carbone CS₂ comme promoteur. Aucune indication n'est donnée au sujet de la stabilité dans le temps de ce système catalytique.

Un catalyseur solide comprenant un acide 12-phosphotungstique supporté sur silice est également décrit par le brevet US 5,420,092. Ce document enseigne, plus généralement, la mise en oeuvre d' un hétéropolyacide combiné à un métal du groupe VIII, mais dans le domaine éloigné de l'isomérisation des paraffines.

Il a maintenant été trouvé un nouveau procédé catalytique pour la préparation de mercaptans à partir de thioéthers et d'hydrogène sulfuré qui met en oeuvre de l'hydrogène dans le flux réactionnel et un catalyseur spécifique. Il présente l'avantage de recourir à des températures plus basses, d'obtenir avec un bon rendement des mercaptans de grande pureté, et de maintenir l'activité élevée du catalyseur au cours du temps.

L'invention a donc pour objet un procédé de fabrication d'un mercaptan à partir d'un thioéther et d'hydrogène sulfuré, caractérisé en ce qu'il est effectué en présence d'hydrogène et d'une composition catalytique comprenant un acide fort et au moins un métal appartenant au groupe VIII de la classification périodique.

La combinaison de l'hydrogène avec cette composition catalytique permet de stabiliser l'activité du catalyseur dans le temps à un niveau élevé et ceci à relativement basse température. Ce résultat est d'autant plus surprenant qu'il est obtenu dans un milieu sulfurant, connu pour empoisonner les sites actifs des catalyseurs.

L'acide fort utilisable dans la composition catalytique est pris dans le groupe comprenant :
- (a) un ou plusieurs hétéropolyacide(s) choisi(s) parmi:
   - (i) un composé de formules : H₃PW₁₂O₄₀,nH₂O, H₄SiW₁₂O₄₀,nH₂O ou H₆P₂W₁₈O₆₂,nH₂O dans lesquelles n est un nombre entier représentant le nombre de molécules d'eau de cristallisation, et (s'agissant d'un produit commercial) est généralement compris entre 0 et 30, de préférence entre 6 et 20 ;
   - (ii) un sel de potassium, rubidium, césium ou d'ammonium d'au moins un composé (i) ou un mélange de tels sels;
- (b) une zircone sulfatée,
- (c) une zircone tungstée,
- (d) une zéolithe, et
- (e) une résine cationique.

L' hétéropolyacide (i) est généralement obtenu par la condensation de 2 ou plus oxoacides différents, tels que l'acide phosphorique, l'acide silicique ou l'acide tungstique. Il est soluble dans l'eau ou dans un solvant organique polaire. Le composé de formule : H₃PW₁₂O₄₀,nH₂O est connu sous la dénomination d'acide 12-phosphotungstique ou 12-tungstophosphorique, et est disponible dans le commerce. Le composé de formule : H₄SiW₁₂O₄₀,nH₂O est connu sous le nom d'acide 12-tungstosilicique ou 12-silicotungstique, et est également disponible dans le commerce. Le composé de formule : H₆P₂W₁₈O₆₂,nH₂O, peut être préparé selon le mode opératoire décrit dans la référence suivante: A. P. Ginsberg, Inorganic Synthesis, Vol 27, Published by J. Wiley & sons (1990) pages 105-107.

L'hétéropolyacide (ii) est un sel obtenu par substitution partielle d'un ou plusieurs protons de l'hétéropolyacide (i) par le cation correspondant. Il est clair pour l'homme du métier qu'une telle substitution ne peut être totale, sans quoi l'acidité serait perdue. Un tel sel est préparé à partir d'une solution de l'hétéropolyacide (i) à laquelle est ajoutée la quantité souhaitée du précurseur de l'alcalin ou de l'ammonium. Le précurseur préféré est le chlorure ou le carbonate correspondant. Le sel précipité est séparé, puis séché dans des conditions douces, de préférence par centrifugation suivie d'une lyophilisation. On peut citer comme référence: N. Essayem, G. Coudurier, M. Fournier, J.C. Vedrine, *Catal. Lett.,* 34 (1995) pages 224-225.

La zircone sulfatée (b) est préparée par imprégnation d'acide sulfurique sur un support d'oxyde de zirconium conformément au procédé décrit dans la référence : F. R. Chen, G. Coudurier, J-F Joly and J.C. Vedrine, *J. Catal.,* 143 (1993) page 617.

La zircone tungstée (c) est préparée par imprégnation d'oxyde de tungstène sur un support d'oxyde de zirconium, conformément au procédé décrit dans le brevet Soled et al. US 5113034.

Selon une première variante de réalisation du procédé selon l'invention, le catalyseur mis en oeuvre dans celui-ci comprend comme acide fort un hétéropolyacide (ii), ou l'un des composés (b), (c), (d) ou (e). Cette variante est préférée car, en raison des propriétés de surface spécifique d'un tel acide fort, ce dernier convient généralement comme support. Il n'est donc pas dans ce cas nécessaire de déposer l'acide fort sur un support.

La composition catalytique comprend dans ce cas:
- de 90 à 99,9 %, de préférence de 98,5 à 99,5 %, en poids d'acide fort, et
- de 0,01 à 10 %, de préférence de 0,05 à 1,5 % en poids de métal du groupe VIII.

Selon une deuxième variante de réalisation, le catalyseur mis en oeuvre comprend comme acide fort un hétéropolyacide (i). Cette variante est préférée en raison d'une activité particulièrement avantageuse du catalyseur dans la réaction de sulfhydrolyse.

La composition catalytique comprend dans ce cas :
- de 10 à 60 %, et de préférence de 25 à 50 % en poids d'acide fort,
- de 0,01 à 10 %, de préférence de 0,1 à 2 % en poids de métal du groupe VIII, et
- de 30 à 80 % de préférence de 48 à 75 % en poids d'un support choisi parmi la silice SiO₂, l'alumine Al₂O₃, l'oxyde de titane TiO₂, la zircone ZrO₂ ou du charbon actif.

Selon un mode de réalisation particulièrement préféré, l'acide fort mis en oeuvre dans le catalyseur est l'acide 12-phosphotungstique, imprégné de préférence sur de la silice.

Le ou les métaux appartenant au groupe VIII de la classification périodique généralement compris dans la composition catalytique mise en oeuvre sont choisis parmi notamment le fer, le cobalt, le nickel, le ruthénium, le rhodium, le palladium, l'osmium, l'iridium, le platine.

On préfère employer un métal du groupe VIII choisi parmi le palladium, le ruthénium et le platine, et tout particulièrement le palladium.

Une composition catalytique particulièrement préférée est celle comprenant : 40 % en poids d'acide 12-phosphotungstique, 1 % de palladium et 59 % de silice.

La composition catalytique mise en oeuvre dans le procédé selon l'invention peut être préparée de façon générale de la manière suivante.

Lorsque l'acide fort utilisé est l'un des composés (i), on procède à :
- (1) un traitement thermique du support sous vide à une température comprise entre 90 et 150°C, de préférence autour de 100°C, puis
- (2) une imprégnation du support ainsi traité avec une solution aqueuse ou organique de pH acide contenant le composé (i) et un précurseur acide du métal du groupe VIII, puis
- (3) un séchage du solide ainsi obtenu, puis
- (4) un traitement par H₂ à une température comprise entre 80 et 300°C, de préférence entre 180 et 250 °C.

Le traitement thermique de l'étape (1) a pour but de désorber l'eau éventuellement adsorbée dans les pores du support.

Dans l'étape (2), on entend par précurseur acide un composé donnant en solution aqueuse un complexe cationique ou anionique dudit métal. Des exemples de tels composés sont, dans le cas du platine: l'hydroxyde de platine tétramine, le chlorure de platine tétramine, le dinitrodiamine-platine (II), ou encore, dans le cas du palladium, le chlorure de palladium, Pd(NH₃)₄Cl₂, (NH₄)₂(PdCl₄). Des exemples de tels composés sont encore, dans le cas du platine : l'acide hexachloroplatinique (encore appelé hexachloroplatinate (IV) d'hydrogène), le tetrachloroplatinate (II) d'ammonium, l'hexachloroplatinate (IV) d'ammonium. La liste des précurseurs acides est donnée précédemment à titre purement illustratif, sans limiter les composés utilisables comme précurseur acide par l'homme du métier.

Dans l'étape (3), le séchage peut être par exemple réalisé en chauffant le support imprégné, éventuellement sous vide, à une température généralement comprise entre la température ambiante et 120 °C, durant un temps allant de 30 minutes à 5 heures.

Le traitement par H₂ de l'étape (4) est avantageusement réalisé sur le catalyseur lorsque ce dernier est placé dans le réacteur de sulfhydrolyse, et a pour but la réduction du précurseur acide en métal du groupe VIII.

Lorsque le catalyseur mis en oeuvre comprend comme acide fort un hétéropolyacide (ii), ou l'un des composés (b), (c), (d) ou (e), il peut être préparé selon le même procédé, à l'exception du fait que le traitement thermique n'est pas obligatoire, et doit même être supprimé ou modifié en fonction des caractéristiques du support.

La composition catalytique décrite précédemment est mise en oeuvre dans le procédé de fabrication de mercaptan selon l'invention qui comprend la réaction d'hydrogène sulfuré (H₂S) sur un thioéther en présence d'hydrogène.

Ce procédé est effectué en phase gaz, dans la mesure où les conditions de température et de pression utilisées sont telles que les réactifs et les produits sont à l'état gazeux.

L'hydrogène introduit dans le procédé à raison d'une quantité correspondant à un rapport molaire H₂S / H₂ compris entre 10 et 200, de préférence entre 50 et 100.

Le thioéther (ou sulfure organique) utilisé comme réactif de départ a pour formule générale :

R-S-R' (I)

dans laquelle R et R', identiques ou différents, représentent un radical alkyle de 1 à 20 atomes de carbone, de préférence de 1 à 12 atomes de carbone, linéaire ou ramifié, ou bien un radical cycloalkyle de 3 à 7 atomes de carbone.

On préfère utiliser comme thioéther de départ un composé de formule (I) dans laquelle R et R' sont identiques. Dans ce cas en effet, il n'est pas nécessaire de procéder à une séparation des thiols obtenus.

Le thioéther encore plus préférentiellement utilisé est le diéthylsulfure (ou éthylthioéther). La réaction de sulfhydrolyse conduit dans ce cas à l'éthylmercaptan (ou éthanethiol).

L'hydrogène sulfuré est introduit dans le procédé en quantité suffisante pour obtenir la conversion du sulfure organique. En général cette quantité correspond à un rapport molaire H₂S / thioéther compris entre 1 et 40, de préférence entre 2 et 30, encore plus préférentiellement entre 2 et 10.

Les réactifs décrits ci-dessus sont mis en contact, en présence d'une charge de la composition catalytique définie précédemment, dans une zone réactionnelle adaptée, dans des condtions réactionnelles propres à produire le thiol désiré.

On préfère mettre en oeuvre le procédé dans un réacteur alimenté en continu par les réactifs, mais un réacteur de type batch peut également être utilisé.

La température de réaction varie selon le thioéther utilisé et le degré de conversion désiré, mais se situe généralement dans un domaine compris entre 50 et 350°C, de préférence entre 150 et 250°C.

La pression à laquelle la réaction est réalisée varie également dans de larges limites. Habituellement, elle se situe entre la pression atmosphérique et 20 bars, de préférence entre 10 et 15 bars.

Le temps de contact est généralement compris entre 1 et 50 s, de préférence entre 10 et 30 s.

Le thioéther mis en oeuvre dans le procédé selon l'invention peut être le sous-produit obtenu dans un procédé de fabrication de thiol par addition de sulfure d'hydrogène sur un alcool ou sur une oléfine, en présence d'un catalyseur et/ou par activation photochimique. Dans cette variante de procédé, on peut ainsi valoriser avantageusement ledit sous-produit.

Les exemples ci-après sont donnés à titre purement illustratifs de l'invention, et ne doivent nullement être interprêtés comme une limitation de celle-ci. Dans ces exemples l'abréviation HPW correspond à l'acide 12-phosphotungstique de formule H₃PW₁₂O₄₀,nH₂O.

### Exemple 1 (Préparatoire): Préparation du catalyseur Pd et HPW supportés sur SiO₂

Pour 200 g de SiO₂, on prépare une solution aqueuse comprenant 6 g de PdCl₂ et 140 g d'HPW (poids exprimé en équivalent acide anhydre, soit avec n égal à 0).

On utilise comme support du catalyseur une silice amorphe ayant une surface spécifique (ou BET) de 315 m².g⁻¹, un diamètre de pores de l'ordre de 12 à 14 nm et un volume poreux de 1,6 cm³.g⁻¹. Ce support est préalablement traité sous vide à une température de 100°C.

L'imprégnation de la solution précédemment obtenue sur le support ainsi traité est réalisée sous vide par aspiration. Une fois la solution imprégnée, le mélange est agité pendant 1 heure à pression atmosphérique.

Le produit obtenu est séché sous vide, à température ambiante, puis est soumis à un traitement par de l'hydrogène à une température de 230°C visant à réduire le palladium.

Le catalyseur obtenu est constitué de 59% en poids de SiO₂, 1% en poids de Pd et 40% en poids d'HPW.

### Exemple 2 : Préparation de l'éthyl mercaptan (CH₃CH₂-SH) à partir du diéthylsulfure (CH₃CH₂-S-CH₂CH₃) :

On utilise un réacteur tubulaire de 25 mm de diamètre présentant une capacité utile de 200 ml chargé de 200 ml de la composition catalytique préparée selon l'exemple 1.
A travers cette charge, on fait passer par heure, 120 g de diéthylsulfure (soit 1 mole), 210 g d'H₂S (soit 5 moles) et 0,8 g d'H₂ (soit 0,08 mole).
La pression dans le réacteur est maintenue à 15 bars et la température est fixée à 235°C.
L'analyse en continu des produits bruts de la réaction montre que la conversion initiale du thioéther est de 52% avec un rendement en éthyl mercaptan de 49,3%.

### Exemple 3 : Préparation de l'éthyl mercaptan (CH₃CH₂-SH) à partir du diéthylsulfure (CH₃CH₂-S-CH₂CH₃) - Evolution de la conversion de l'éthyl mercaptan en fonction du temps

On répète l'exemple 2 en poursuivant la réaction de sulfhydrolyse pendant 6 jours avec la même charge de composition catalytique, et en procédant périodiquement (en fonction du temps exprimé en jours) à la mesure de la conversion du diéthyl sulfure (DES).

Les résultats sont rassemblés dans le tableau suivant.

**Tableau 1**

| **Temps (jours)** | **Conversion du DES (en %)** |
|---|---|
| 1 | 54 |
| 3 | 56 |
| 4 | 55 |
| 5 | 56 |
| 6 | 57 |

Le tableau 1 montre que le système catalytique préparé à l'exemple 1 et utilisé en présence d'hydrogène selon le procédé de l'invention a une bonne stabilité au cours du temps.

## Revendications

1. Procédé de fabrication d'un mercaptan à partir d'un thioéther et d'hydrogène sulfuré, **caractérisé en ce qu'**il est effectué en présence d'hydrogène et d'une composition catalytique comprenant un acide fort et au moins un métal appartenant au groupe VIII de la classification périodique.

2. Procédé selon la revendication 1, **caractérisé en ce que** l'acide fort est pris dans le groupe comprenant:
- (a) un ou plusieurs hétéropolyacide(s) choisi(s) parmi:
- (i) un composé de formules : H₃PW₁₂O₄₀,nH₂O, H₄SiW₁₂O₄₀,nH₂O ou H₆P₂W₁₈O₆₂,nH₂O dans lesquelles n est un nombre entier représentant le nombre de molécules d'eau de cristallisation, et est compris entre 0 et 30, de préférence entre 6 et 20 ;
- (ii) un sel de potassium, rubidium, césium ou d'ammonium d'au moins un composé (i) ou un mélange de tels sels;
- (b) une zircone sulfatée,
- (c) une zircone tungstée,
- (d) une zéolithe, et
- (e) une résine cationique.

3. Procédé selon la revendication 2, **caractérisé en ce que** le catalyseur mis en oeuvre comprend comme acide fort un hétéropolyacide(ii), ou l'un des composés (b), (c), (d) ou (e).

4. Procédé selon la revendication 3, **caractérisé en ce que** la composition catalytique comprend :
- de 90 à 99,9 %, de préférence de 98,5 à 99,5 %, en poids d'acide fort, et
- de 0,01 à 10 %, de préférence de 0,05 à 1,5 % en poids de métal du groupe VIII.

5. Procédé selon la revendication 2, catactérisé en ce que le catalyseur mis en oeuvre comprend comme acide fort un hétéropolyacide (i).

6. Procédé selon la revendication 5, catactérisé en ce que la composition catalytique comprend :
- de 10 à 60 %, et de préférence de 25 à 50 % en poids d'acide fort,
- de 0,01 à 10 %, de préférence de 0,1 à 2 % en poids de métal du groupe VIII, et
- de 30 à 80 % de préférence de 48 à 75 % en poids d'un support choisi parmi la silice SiO₂, l'alumine Al₂O₃, l'oxyde de titane TiO₂, la zircone ZrO₂ ou du charbon actif.

7. Procédé selon l'une des revendications 5 ou 6, **caractérisé en ce que** l'acide fort mis en oeuvre dans le catalyseur est l'acide 12-phosphotungstique, imprégné de préférence sur de la silice.

8. Procédé selon l'une des revendications 1 à 7, **caractérisé en ce que** le ou les métaux appartenant au groupe VIII de la classification périodique sont choisis parmi le fer, le cobalt, le nickel, le ruthénium, le rhodium, le palladium, l'osmium, l'iridium, le platine.

9. Procédé selon la revendication 8, **caractérisé en ce que** le ou les métaux sont choisis parmi le palladium, le ruthénium et le platine.

10. Procédé selon l'une des revendications 8 ou 9, **caractérisé en ce que** le métal est le palladium.

11. Procédé selon l'une des revendications 1 ou 5 à 10, **caractérisé en ce que** la composition catalytique comprend : 40 % en poids d'acide 12-phosphotungstique, 1 % de palladium et 59 % de silice.

12. Procédé selon l'une des revendications 1 à 11, **caractérisé en ce que** l'hydrogène est introduit à raison d'une quantité correspondant à un rapport molaire H₂S / H₂ compris entre 10 et 200, de préférence entre 50 et 100.

13. Procédé selon l'une des revendications 1 à 12, **caractérisé en ce que** le thioéther utilisé a pour formule générale :
R-S-R' (I)
dans laquelle R et R', identiques ou différents, représentent un radical alkyle de 1 à 20 atomes de carbone, de préférence de 1 à 12 atomes de carbone, linéaire ou ramifié, ou bien un radical cycloalkyle de 3 à 7 atomes de carbone.

14. Procédé selon l'une des revendications 1 à 13, **caractérisé en ce que** l'hydrogène sulfuré est introduit dans une quantité correspondant à un rapport molaire H₂S /thioéther compris entre 1 et 40, de préférence entre 2 et 30, encore plus préférentiellement entre 2 et 10.

## Claims

1. Process for the manufacture of a mercaptan from a thioether and hydrogen sulphide, **characterized in that** it is carried out in the presence of hydrogen and of a catalytic composition comprising a strong acid and at least one metal belonging to Group VIII of the Periodic Table.

2. Process according to Claim 1, **characterized in that** the strong acid is taken from the group consisting of:
- (a) one or more heteropolyacid(s) chosen from:
- (i) a compound of formula: H₃PW₁₂O₄₀·nH₂O, H₄SiW₁₂O₄₀.nH₂O or H₆P₂W₁₈O₆₂·nH₂O, in which n is an integer representing the number of molecules of water of crystallization and is between 0 and 30, preferably between 6 and 20;
- (ii) a potassium, rubidium, caesium or ammonium salt of at least one compound (i) or a mixture of such salts;
- (b) a sulphated zirconia,
- (c) a tungstated zirconia,
- (d) a zeolite, and
(e) a cationic resin.

3. Process according to Claim 2, **characterized in that** the catalyst employed comprises, as strong acid, a heteropolyacid (ii) or one of the compounds (b), (c), (d) or (e).

4. Process according to Claim 3, **characterized in that** the catalytic composition comprises:
- from 90 to 99.9%, preferably from 98.5 to 99.5%, by weight of strong acid, and
- from 0.01 to 10%, preferably from 0.05 to 1.5%, by weight of metal from Group VIII.

5. Process according to Claim 2, **characterized in that** the catalyst employed comprises, as strong acid, a heteropolyacid (i).

6. Process according to Claim 5, **characterized in that** the catalytic composition comprises:
- from 10 to 60%, preferably from 25 to 50%, by weight of strong acid,
- from 0.01 to 10%, preferably from 0.1 to 2%, by weight of metal from Group VIII, and
- from 30 to 80%, preferably from 48 to 75%, by weight of a support chosen from silica SiO₂, alumina Al₂O₃, titanium oxide TiO₂ zirconia ZrO₂ or active charcoal.

7. Process according to either of Claims 5 and 6, **characterized in that** the strong acid employed in the catalyst is 12-phosphotungstic acid, preferably impregnated on silica.

8. Process according to one of Claims 1 to 7, **characterized in that** the metal or metals belonging to Group VIII of the Periodic Table are chosen from iron, cobalt, nickel, ruthenium, rhodium, palladium, osmium, iridium and platinum.

9. Process according to Claim 8, **characterized in that** the metal or metals are chosen from palladium, ruthenium and platinum.

10. Process according to either of Claims 8 and 9, **characterized in that** the metal is palladium.

11. Process according to one of Claims 1 or 5 to 10, **characterized in that** the catalytic composition comprises 40% by weight of 12-phosphotungstic acid, 1% of palladium and 59% of silica.

12. Process according to one of Claims 1 to 11, **characterized in that** the hydrogen is introduced in the proportion of an amount corresponding to an H₂S/H₂ molar ratio of between 10 and 200, preferably between 50 and 100.

13. Process according to one of Claims 1 to 12, **characterized in that** the thioether used has the general formula:
R-S-R' (I)
in which R and R', which are identical or different, represent a linear or branched alkyl radical of 1 to 20 carbon atoms, preferably of 1 to 12 carbon atoms, or else a cycloalkyl radical of 3 to 7 carbon atoms.

14. Process according to one of Claims 1 to 13, **characterized in that** the hydrogen sulphide is introduced in an amount corresponding to an H₂S/thioether molar ratio of between 1 and 40, preferably between 2 and 30, more preferably still between 2 and 10.

## Patentansprüche

1. Verfahren zur Herstellung eines Mercaptans aus einem Thioether und Schwefelwasserstoff, **dadurch gekennzeichnet, daß** man es in Gegenwart von Wasserstoff und einer eine starke Säure und wenigstens ein zur Gruppe VIII des Periodensystems der Elemente zählendes Metall enthaltenden katalytischen Zusammensetzung durchführt.

2. Verfahren nach Anspruch 1, **dadurch gekennzeichnet, daß** die starke Säure aus der folgenden Gruppe stammt:
- (a) eine oder mehrere Heteropolysäuren ausgewählt aus:
- (i) einer Verbindung der Formel: H₃PW₁₂O₄₀.nH₂O, H₄SiW₁₂O₄₀·nH₂O oder H₆P₂W₁₈O₆₂·nH₂O, wobei n für eine ganze Zahl steht, die die Anzahl von Kristallwassermolekülen wiedergibt und zwischen 0 und 30, vorzugsweise zwischen 6 und 20, liegt;
- (ii) ein Kalium-, Rubidium-, Caesium- oder Ammoniumsalz wenigstens einer Verbindung (i) oder eine Mischung solcher Salze;
- (b) ein sulfatiertes Zirconiumdioxid,
- (c) ein wolframiertes Zirconiumdioxid,
- (d) ein Zeolith und
- (e) ein kationisches Harz.

3. Verfahren nach Anspruch 2, **dadurch gekennzeichnet, daß** der verwendete Katalysator als starke Base eine Heteropolysäure (ii) oder eine der Verbindungen (b), (c), (d) oder (e) enthält.

4. Verfahren nach Anspruch 3, **dadurch gekennzeichnet, daß** die katalytische Zusammensetzung:
- 90 bis 99,9 Gew.-%, vorzugsweise 98,5 bis 99,5 Gew.-%, an starker Säure und
- 0,01 bis 10 Gew.-%, vorzugsweise 0,05 bis 1,5 Gew.-%, an Metall aus der Gruppe VIII
enthält.

5. Verfahren nach Anspruch 2, **dadurch gekennzeichnet, daß** der verwendete Katalysator als starke Säure eine Heteropolysäure (i) enthält.

6. Verfahren nach Anspruch 5, **dadurch gekennzeichnet, daß** die katalytische Zusammensetzung:
- 10 bis 60 Gew.-%, vorzugsweise von 25 bis 50 Gew.-%, an starker Säure,
- 0,01 bis 10 Gew.-%, vorzugsweise von 0,1 bis 2 Gew.-%, an Metall aus der Gruppe VIII, und
- 30 bis 80 Gew.-%, vorzugsweise von 48 bis 75 Gew.-%, eines aus Siliciumdioxid SiO₂, Aluminiumoxid Al₂O₃, Titandioxid TiO₂, Zirconiumoxid ZrO₂ oder Aktivkohle ausgewählten Trägers
enthält.

7. Verfahren nach Anspruch 5 oder 6, **dadurch gekennzeichnet, daß** es sich bei der im Katalysator verwendeten starken Säure um 12-Phosphowolframsäure, vorzugsweise imprägniert auf Siliciumdioxid, handelt.

8. Verfahren nach einem der Ansprüche 1 bis 7, **dadurch gekennzeichnet, daß** das bzw. die zur Gruppe VIII des Periodensystems der Elemente zählende Metall/zählenden Metalle ausgewählt ist/sind aus Eisen, Cobalt, Nickel, Ruthenium, Rhodium, Palladium, Osmium, Iridium und Platin.

9. Verfahren nach Anspruch 8, **dadurch gekennzeichnet, daß** das Metall bzw. die Metalle ausgewählt ist/sind aus Palladium, Ruthenium und Platin.

10. Verfahren nach Anspruch 8 und 9, **dadurch gekennzeichnet, daß** es sich bei dem Metall um Palladium handelt.

11. Verfahren nach einem der Ansprüche 1 oder 5 bis 10, **dadurch gekennzeichnet, daß** die katalytische Zusammensetzung 40 Gew.-% 12-Phosphowolframsäure, 1 Gew.-% Palladium und 59 Gew.-% Siliciumdioxid enthält.

12. Verfahren nach einem der Ansprüche 1 bis 11, **dadurch gekennzeichnet, daß** der Wasserstoff in einer Menge eingebracht wird, die einem H₂S/H₂-Molverhältnis zwischen 10 und 200, vorzugsweise zwischen 50 und 100, entspricht.

13. Verfahren nach einem der Ansprüche 1 bis 12, **dadurch gekennzeichnet, daß** der verwendete Thioether die allgemeine Formel:
R-S-R' (I)
aufweist, wobei R und R', die gleich oder verschieden sind, für einen geradkettigen oder verzweigten Alkylrest mit 1 bis 20 Kohlenstoffatomen, vorzugsweise 1 bis 12 Kohlenstoffatomen, oder einen Cycloalkylrest mit 3 bis 7 Kohlenstoffatomen, stehen.

14. Verfahren nach einem der Ansprüche 1 bis 13, **dadurch gekennzeichnet, daß** der Schwefelwasserstoff in einer Menge eingebracht wird, die einem H₂S/Thioether-Molverhältnis zwischen 1 und 40, vorzugsweise zwischen 2 und 30, besonders bevorzugt zwischen 2 und 10, entspricht.
